# EUROPEAN PATENT SPECIFICATION

(11) **EP 0 632 082 B1**
(45) Date of publication and mention of the grant of the patent: **02.04.1997**
(21) Application number: 93870124.0
(22) Date of filing: 29.06.1993
(51) Int. Cl.: C08G 65/32, A61K 47/48

(54) **Preparation of activated carbamates of poly(alkylene glycol) and their use**
Herstellung von aktivierten Carbamaten von Polyalkylenglycol und ihre Verwendung
Préparation de carbamates actifs de polyalkylène glycol et leur utilisation

(43) Date of publication of application: 04.01.1995
(73) Proprietor: "HEYLECINA", Société Anonyme, B-1357 Hélecine (BE)
(72) Inventor: Looze, Yvan, Protein Chemistry Unit, Faculty of, B-1050 Brussels (BE)
(74) Representative: Schmitz, Yvon

(56) References cited:
- EP-A- 0 317 780
- EP-A- 0 605 963
- DE-A- 2 805 239
- FR-A- 2 253 011
- J. BIOACTIVE AND COMPATIBLE POLYMERS vol. 4 , January 1989 pages 17 - 24 YOSHINGA K. ET AL 'Effects of coupling chemistry on the activity of poly(ethylene glycol) modified alkaline phosphatase'
- EUR. POLYM. J. vol. 19, no. 12 , 1983 pages 1177 - 1183 ZALIPSKY S. ET AL 'Attachment of drugs to polyethylene glycols'

## Description

### FIELD OF THE INVENTION

The present invention relates to chemical modification of polypeptides by means of covalent attachment by strands of poly(alkylene glycol) in order to improve their delivery of drugs.

### BACKGROUND OF THE INVENTION

The potential values of peptides and proteins as therapeutics has been recognized for a long time. By the same time, their delivery, still today, represents a challenge.

Historically, before the development of large-scale manufacturing processes for human recombinant proteins, most proteins and polypeptides that were used for therapy were derived from non-human sources.

Unfortunately, repeated administration of such non-human proteins often induced the development of immune responses that resulted in hypersensitivity or even death. Furthermore, the development of an immune response was also associated with increased protein clearance, thus necessitating the use of higher doses to maintain therapeutical efficiency.

As a consequence, it often resulted in a viscious cycle of higher doses inducing immune response, increasing the clearance and thus giving rise to lack of efficacy.

It was believed that the use of human proteins such as those produced by the recombinant technology would eleminate these problems.

An increasingly growing number of recombinant human proteins are now commercially available. Also, a number of the recombinant proteins have already been approved for use by the Food and Drug Administration (FDA). Nevertheless, as evaluation of these proteins was in progress, it became evident that the hope that recombinant technology would solve all the problems associated with the therapeutical use of proteins was not realized. The difficulty encountered with recombinant interleukine-2 (rIL-2) is a typical example.

Human interleukine-2 (IL-2), a single polypeptide chain containing 133 aminoacids, is glycosylated on threonine in position 3, while rIL-2, produced in E.Coli is unglycosylated (R.Devos et al, Nucleic Acids Res., 1983, **11**:4307 and S.A.Rosenberg et al, Science, 1984, **223**:1412).

As a result of the lack of carbohydrate moiety, rIL-2 is poorly soluble in aqueous solutions at physiological pH and has a very short circulatory half-life (initial half-life of 7 to 13 minutes and final half-life of 70 to 85 minutes - M.T.Lotze et al, J.Immunol., 1985, **135**:2865.- M.W.Konrad et al, Cancer Res., 1990, **50**:2009.).

Furthermore, antibodies to rIL-2 have been found in patients undergoing clinical trials with the recombinant cytokine (M. Allegretta et al, J.Clin.Immunol., 1986, **6**:481.; M.B. Atkins et al, J. Clin. Oncol., 1986, **4**:1380.; R.L. Krigel et al, Cancer Res., 1988, **48**:3875.). To overcome these problems, higher doses have been used and long-term i.v. administration of rIL-2 has been tried with success; antitumoral effects were indeed obtained. However, the number of responding patients was limited and the treatment was not exempt of serious toxicity (S.A.Rosenberg et al, N.Engl.J.Med., 1987, **316**:889.).

It should also be mentioned that some potentially useful enzymes (e.g. uricase which reduces the level of uric acid in plasma and urine) for human therapy are not produced by human cells.

Thus, the delivery of polypeptides either of non-human origins and even of human origin needs to be further investigated in order to confer pharmacological efficacy to this material when used as pharmaceuticals.

The chemical attachment of poly(alkylene glycol)(PAG) to polypeptides is now recognized as a method of choice for conferring to the modified polypeptides many new and interesting properties with regard to their use as drugs.

As a general rule, PAG-modified polypeptides exhibit increased stability, increased resistance to the proteolytic inactivation, increased circulating lives, decreased to non-existent antigenicity and immunogenicity and low toxicity.

As a consequence of the potential usefulness of all these conferred properties, the covalent attachment of PAG chains to a variety of proteins of therapeutical interest has since been attempted and are nowadays evaluated clinicaly.

A **PAG**-modified form of adenosine deaminase (**ADAGEN**™) has even received approval by the **FDA** for marketing for the treatment of severe combined immunodeficiency disease associated with adenosine deaminase deficiency.

In the case of rIL-2 which was discussed previously, the chemical grafting of 2 to 3 poly(ethylene glycol) (PEG) chains (7 kDa each) resulted in a compound (PEG-rIL-2) with enhanced solubility and with a 10 to 20 fold prolonged circulatory half-life (M.J. Knauf et al, J. Biol. Chem., 1988, **263**:15064.- F.J. Meyer et al, Clin. Pharmacol. Ther., 1991, **49**:307.).

Furthermore, i.v. administered PEG-rIL-2 showed a significantly higher antitumoral activity than rIL-2 (F.J. Meyer et al, Loc. cit.; R.J. Zimmerman et al, Cancer Res., 1989, **49**:6521.-Y.Shih et al, Eur. J. Immunol., 1992, **22**:127.- V. Mattijssen et al, Int. J. Cancer, 1992, **51**:812.- J.C. Yang et al, Lymphokine Cytokine Res., 1992, **10**:475.) combined with a reduced immunogenicity (N.V. Katre, J.Immunol., 1990, **144**:209.)

Since its introduction as early as 1977 (A. Abuchowski et al, 1977, J. Biol. Chem., **152**:3578.), the technology making use of **PAG** has been evaluated in various medical fields such as enzymes replacement therapy (S. Chaffee et al, 1992,
J. Clin. Investig., **89**:1643.), cancer immunotherapy (F.J. Meyers et al, 1991, Loc.cit.;
V. Mattijssen et al, 1992, Loc.cit.) and allergy hyposensitizing treatments (U. Müller et al, 1987, J. Allergy Clin. Immunol., **80**:252.- H. Mosbech et al, 1990, Allergy, **45**:130.)

Furthermore, it is expected, from the observation that modification of protein's epitopes with PAG induced immunological tolerance towards the native epitopes, that the PAG technology might show promise in the area of autoimmune diseases (A.M. Schon, 1991, Advanced Drug Delivery Rev., **6**:203.-M.Z. Atassi et al, 1992, Proc. Natl. Acad. Sci. U.S.A., **89**:5852.).

Finally, mention should be made of the observation that the covalent modification of proteins with PAG chains may be followed on by an increased and/or selective uptake of these polypeptides by various types of cells and/or tissues (P. Caliceti et al, 1989, Il Farmaco, **44**:711.; J.S. Beckman et al, 1988, J. Biol. Chem., **263**:6884.; A.V. Kabanov et al, 1992, J. Controlled Release, **22**:141.).

Although the mechanism of this process remains debatable, several lines of evidence suggest that PAG facilitates binding to cell membranes; this step being then followed by endocytosis of the PAG-polypeptide conjugate (J.S. Beckman et al, Loc.cit.).

Evidence that PEG interacts with cell membranes is now well documented since the discovery of the PEG-induced fusion of cells and liposomes (K.N. Kao et al, 1974, Planta, **120**:215.; Q. Ahkong et al, 1975, Nature, **253**:194; G. Fontecorvo, 1975, Somat. Cell Genet, **1**:397.; M. Yamazaki and T. Ito, 1990, Biochemistry **29**:1309.; L.T. Boni et al, 1984, Biochim. Biophys. Acta, **775**:409.; G.D. Bittner et al, 1986, Brain Res., **367**:351.; T.L. Krause and G.D. Bittner, 1990, Proc. Natl. Acad. Sci. U.S.A., **87**:1471.; N. Geron and H.Meiri, 1985, Biochim. Biophys. Acta, **819**:258.).

Also, the interpretation of some two-phase partitioning results, noteworthy, requires the concept that PEG interacts with cell membranes (H. Walter et al, 1992,J. Chromatog., **609**:219.). It may be expected from all these observations that the use of PAG might be made to target polypeptide drugs inside selected cells and/or tissues.

PAG's are linear, hydrophobic, uncharged and flexible polymers which are commercially available in a variety of molecular weights.

These polymers are not toxic and generally recognized as safe. In particular, monomethoxy poly(ethylene glycol) (mPEG) is approved by the FDA as a vehicle or base for a number of pharmaceutical preparations and has a low order of toxicity in oral, parenteral and epidermal applications (C.B.Shaffer and F.M. Critchfield, 1947, J.Am. Pharm. Assoc., **36**:157.; C.P. Carpenter and C.B. Shaffer, 1952, J. Am. Pharm. Assoc., **41**:27.; H.F. Smyth et al, 1950, J. Am. Pharm. Assoc., **39**:349.)

When administrated intravenously to human, mPEG of molecular weight from 1 to 6 kDa are readily excreted, mainly via the kidney (C.B. Shaffer and F.M. Critchfield, 1947, Loc.cit.).

The low toxicity of PAG which can explain the low toxicity of the polypeptides to which it is covalently attached represents a major advantage with the inherent drawback that PAGs are devoid of any chemical reactivity.

As a consequence, to effect covalent attachment of PAG to polypeptides, the hydroxyl end-groups of the PAG polymers must first be converted into reactive functional groups.

This process is frequently referred to as "activation" and the products are called "activated" or "derivatized" or "functionalized" PAGs.

mPAG's derivatives, capped on one end with a functional group have been used in most cases although the methods used for activation of PAG or mPAG do not significantly differ from a strictly chemical point of view.

The chemical methods to provided activated PAG and which represent the key step for the obtention of suitable PAG-polypeptide conjugates have been the subject of intense efforts in the past deceny.

Polymeric derivatives for the selective attachment of PEG to arginine side chains of polypeptides were introduced to serve as carriers during semisynthesis of peptides in aqueous solutions (C.S. Pande et al, 1980, Proc. Natl. Acad. Sci. U.S.A.; **77**:895.; J.D. Glass et al, 1981 in : Peptides Structure and Function, (D. Rich ed) p209 Pierce Chemical Co, Rockford, IL; J.D. Glass et al, 1979, in : Peptides 1978 (I.Z. Siemion and G. Kupryszewski, eds) p235, Wroclav University Press, Poland).

The reagents were readily obtainable by reaction of camphorquinone-10-sulfonyl chloride with mPEG, mPeg-NH₂, or preferably with the norleucine ester of the polymer. 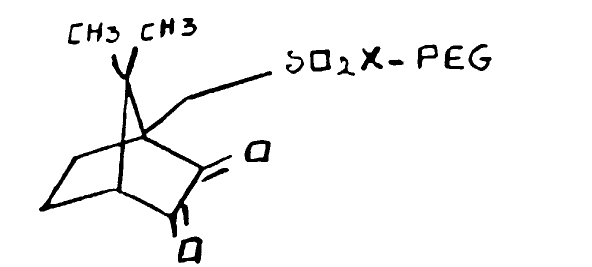 X = O, NH or N → O

### DERIVATIVE D1

The modified peptides were formulated in borate buffer at pH 9.0 at 37°C.

They were stable to a variety of acidic and nucleophilic reagents, including hydroxylamine, the recommended agent for the cleavage of cyclohexanedione-arginine adducts. Active peptides could be released from the polymer carrier by O-phenylenediamine. When norleucine was incorporated as a spacer arm between the camphorquinone moiety and the polymer, the extent of arginine modification was conveniently determined by amino acids analysis of hydrolysates of the PEG-peptides.

A variety of proteins were also modified with **DERIVATIVE D2**, a mPEG-5kDa analog of phenylglyoxal (Eur. Patent Appl., **0.340.741-** 1989 - to A. Sano, H. Maeda, Y. Kai and K. Ono), a well-known reagent for modification of guanidino groups (side chain function of arginine residues). The synthesis of **DERIVATIVE D2** was performed according to the reactions depicted on Scheme 01.

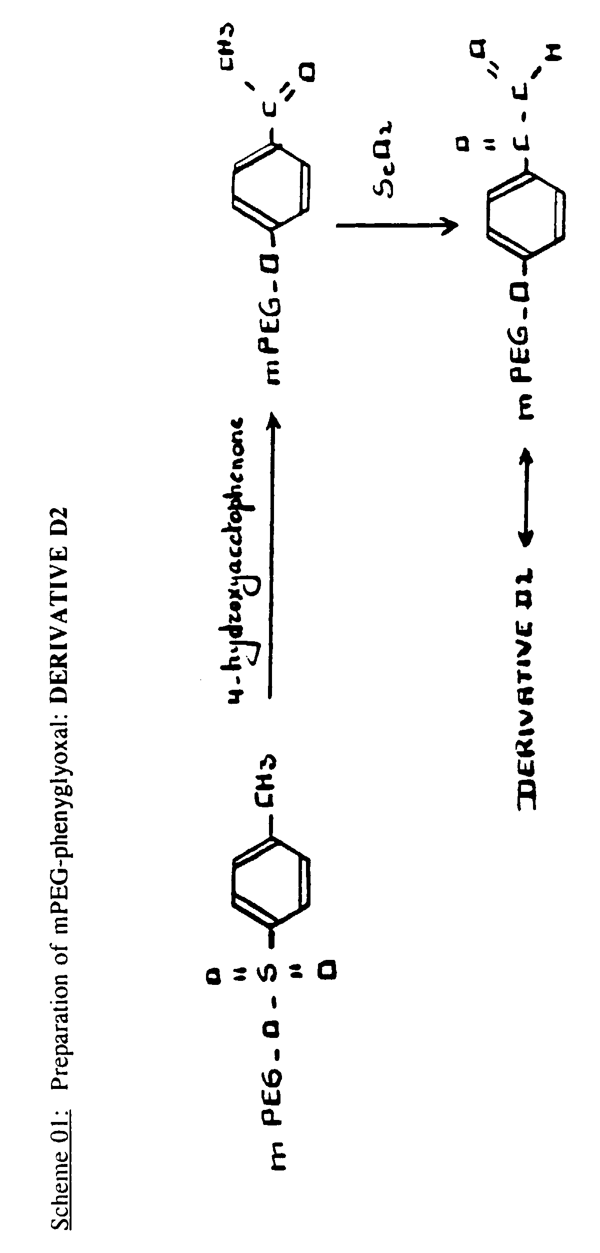

Readily obtainable mPEG-tosylate (S. Zalipsky et al, 1987, Int. J. Peptide Protein Res., **30**:740.; J.M. Harris et al, 1984, J. Polym. Sci. Polym. Chem. Ed., **22**:341.) was subjected to nucleophilic displacement by 4-hydroxyacetophenone, which was then oxidized by selenium dioxide to yield mPEG-phenylglyoxal.

Attachment of **DERIVATIVE D2** to proteins through arginyl residues proceeded at pH range 5.5-9.3, at room temperature, and was measured by the decrease of arginine content in hydrolysates of modified polypeptides. The conjugate of **DERIVATIVE D2** was claimed to possess very good stability and biological activity.

Maleimide-containing reagents are effective modifiers of free cysteinyl residues of polypeptides (M.Z. Atassi, 1977, in : Immunochemistry of Proteins-M.Z. Atassi, Ed. Vol 1, p1, Plenium Press, New York). Preparation of **DERIVATIVE D3** bearing maleimido functional groups was reported using accessible PEG-NH₂ (S. Zalipsky et al, 1983, Eur. Polym. J., **19**:1177.; A. Buckmann et al, 1981, Makromol. Chem., **182**:1379) and active esters of 6-maleimidohexanoic acid (R.J. Goodson and N.V. Katre, 1990, Biotechnology, **8**:343) and 3-maleimidopropionic acids (S. Romani et al, 1984, in : Chemistry of Peptides and Proteins - W. Voelter, E. Bayer, Y.A. Oychinnikov and E. Wunsch, Eds - Vol 2, p.29, Walter de Gruyter, Berlin) as starting material (Scheme 02).

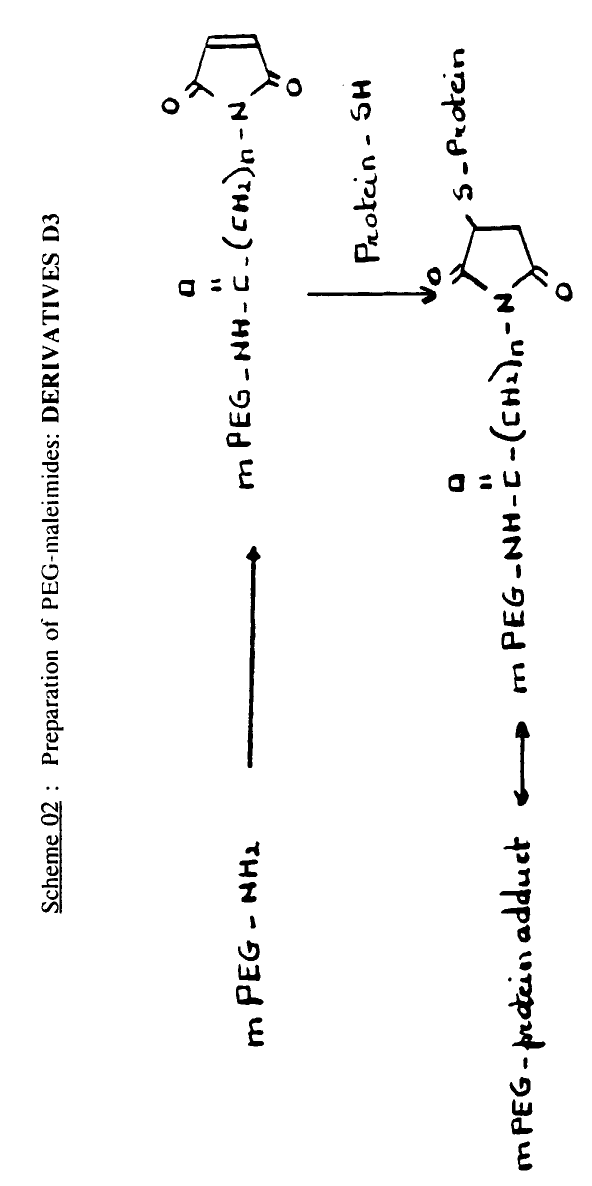

A mutant protein of interleukine-2, in which cysteine at position 3 replaced the naturally occuring glycosylated threonine residue, was coupled with **DERIVATIVE D3** (n=5) to produce a well defined, fully bioactive PEG-polypeptide (R.J. Goodson and N.V. Katre, 1990, Loc.cit.). **DERIVATIVE D3** (n=2) was used for selective entrapment of free thiol-containing peptides on the polymer, in order to simplify purification of unsymmetrical cystine-peptides from a reaction mixture (S. Romani et al, 1984, Loc.cit.).

Amino-PEG was reacted with 1-ethyl-3-(3-dimethylaminopropyl)carbodiimide-activated carboxyl groups of trypsin and other proteins (U.S. Patent 4, 179, 337, (1979) to F.F. Davis, T. Van Es and N.C. Palczuk).

The selectivity of such reaction is rather poor, due to the fact that amino-PEG has similar reactivity to the lysyl residues of proteins. In another variation of this reaction, p-aminobenzylether of PEG was coupled to carboxyl groups of D-glucose 6-phosphate dehydrogenase by treatment with 1-ethyl-3-(3-aminopropyl)carbodiimide (A. Pollak and G.M. Whitesides, 1976, J. Am. Chem. Soc., **98**:289).

The crude conjugate retained 22% of the original activity. The aromatic amino groups on the polymer have a lower pKa than the amino groups of lysine residues and therefore would be expected to exhibit higher reactivity towards activated carboxyls under the acidic conditions of such reactions (pH 4.8-6.0). In both types of modification involving aliphatic and aromatic amino-PEGs, the composition of the conjugates was not determined.

In a recent report, bilirubin oxidase was modified by a two-step protocol (M. Kimura et al, 1988, Proc. Soc. Exp. Biol. Med., **188**:364).

First, 1,4-diaminobutane was coupled to the carboxyl groups using water-soluble carbodiimide.

Then, both the newly introduced amino groups and the original amines of the protein were modified with cyanuric chloride.

The activity of the PEG-bilirubin oxidase conjugate was approximately 30% that of the native enzyme.

Unfortunately, the composition of the conjugate was not reported. When using carbodiimide-mediated coupling of an appropriate nucleophile to a protein, one has to be aware of possible side reactions such as modification of tyrosyl and cysteinyl residues as well as the formation of N-acylurea derivatives (M.Z. Atassi, 1977, Loc.cit.).

In the original work of Davis and co-workers (Abuchowsky et al, 1977, Loc.cit.) 2,4,6-trichloro-s-triazine (cyanuric chloride) was reacted with the primary alcohol groups on PEG so that one of the chlorides was displaced from the triazine ring and one of the remaining chlorides was used for subsequent reaction with the amino groups of the polypeptide as shown in Scheme 03.

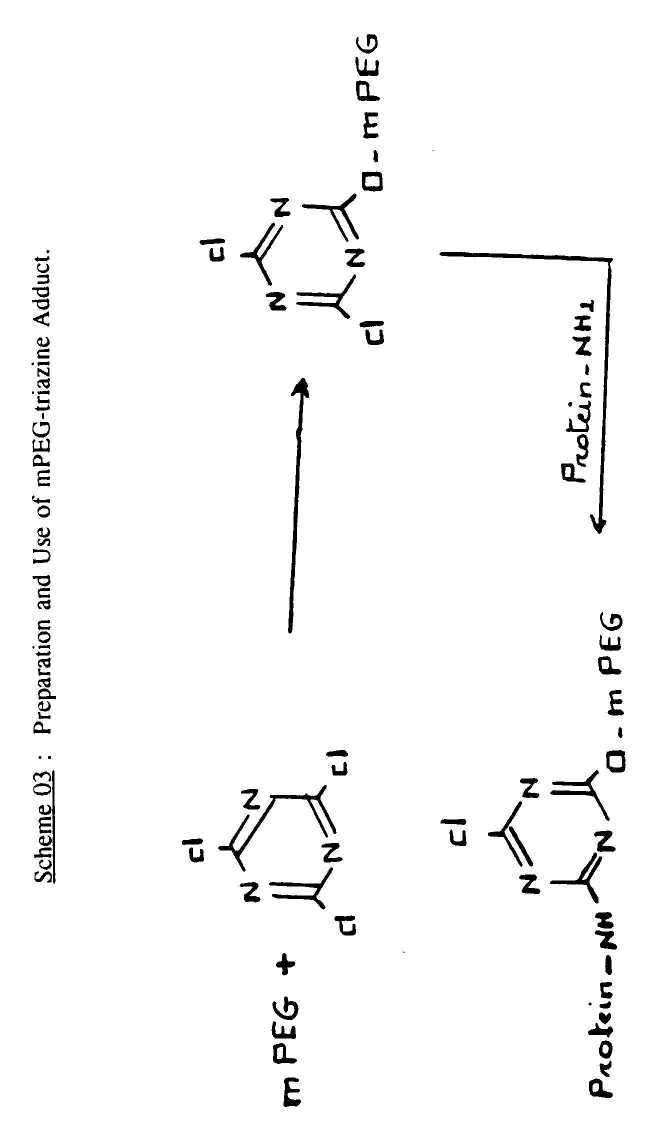

This approach was adopted later on by many other investigators and this method has remained one of the most popular for a long time.

Although this approach provides a very effective one-step activation of PAG, it suffers from various disadvantages such as toxicity of cyanuric chloride and excessive reactivity of the PEG-triazine adduct towards nucleophilic functional groups other than amines (e.g., cysteinyl and tyrosyl residues). Consequently, modification of some proteins with the PEG-triazine adducts is often accompanied by a substantial loss of biological activity (Y. Ashihara et al, 1978, Biochem. Biophys. Res. Commun., **93**:385.; K.J. Wieder et al, 1979, J. Biol. Chem., **254**:12579.; S. Davis et al, 1981, Clin. Exp. Immunol., **46**:649.; K. Yashinaga and J.M. Harris, 1989, J. Bioact. Compatible Polym., **4**:17.).

Furthermore, all the approaches discussed so far suffer in common from the existence of remaining rings (some of them are aromatic) linking the polypeptide to the PAG strands.

Such structures, present in the PAG-polypeptide conjugates, might be suspected to create some toxic problems.

The shortcomings of the activation methods discussed here so far were overcome by using PEG-succinimidyl succinate (**SS-PEG, DERIVATIVE D4**). This form of activated PEG was first used for crosslinking of proteins (U.S.Patent 4,101,380 to M. Rubinstein - 1978) and synthesis of peptides substituted by PEG chains at their terminals (M. Jappich and P.L. Luisi, 1979, Makromol. Chem., **180**:1381) but was later adapted for preparation of mPEG-protein conjugates (A. Abuchowski et al, 1984, Cancer Biochem. Biophys., **7**:175).

**DERIVATIVE D4** is usually prepared by succinylation of the terminal hydroxyl groups of PEG followed by dicyclohexylcarbodiimide-mediated condensation with N-hydroxysuccinimide. It reacts with proteins within a short period of time under mild conditions (30 minutes; pH <7.8; 25°C), producing extensively modified proteins with well-preserved biological activities.

Unfortunately, the ester linkage between the PEG polymer and the succinic ester residue has a limited stability in aqueous media (U.S. Patent 4,670,417-1987 to K. Iwasaki, Y. Iwashita and T. Okami) which causes slow hydrolytic cleavage of PEG chains from SS-PEG-modified polypeptides under physiological conditions.

### SUCCINIMIDYL-PEG DERIVATIVES

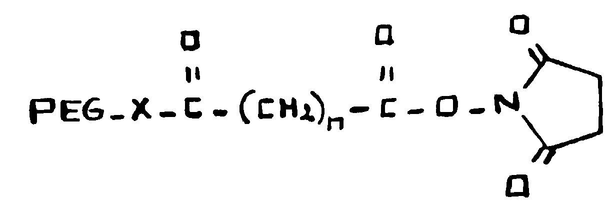

D4 M = 2, X = O
D5 M = 3, X = O
D6 M = 2, X = NH

Substitution of the succinate moiety in derivative D4 by glutarate produced an activated form of the polymer (**DERIVATIVE D5**) very similar to SS-PEG but with a slightly improved resistance to hydrolysis of the ester linkage (V.N. Katre et al, 1987, Proc. Natl. Acad. Sci. U.S.A., **84**:1487).

Also, replacement of the aliphatic ester in SS-PEG by an amide bond to yield **DERIVATIVE D6** improves the stability of the reagent and its conjugates even further (U.S. Patent 4,670,417. Loc.cit.). Unfortunately, preparation of **DERIVATIVE D6**, starting from hydroxyl terminated mPEG, involves 4 to 5 synthesis steps (E. Boccu et al, 1983, Z. Naturforsch.**, 38C**:94.).

Formation of urethane (carbamate) linkages between the amino groups of a protein and PAG are completely stable under a variety of physiological conditions (D.Larwood and F. Szoka, 1984, J. Labelled Compd. Radiopharm., **21**:603.).

The direct (without spacer arm) attachment of mPEG chains to polypeptides through carbamate linkages was first accomplished using imidazoyl formate derivatives of PEG (**DERIVATIVE D7**) as depicted in SCHEME 04.

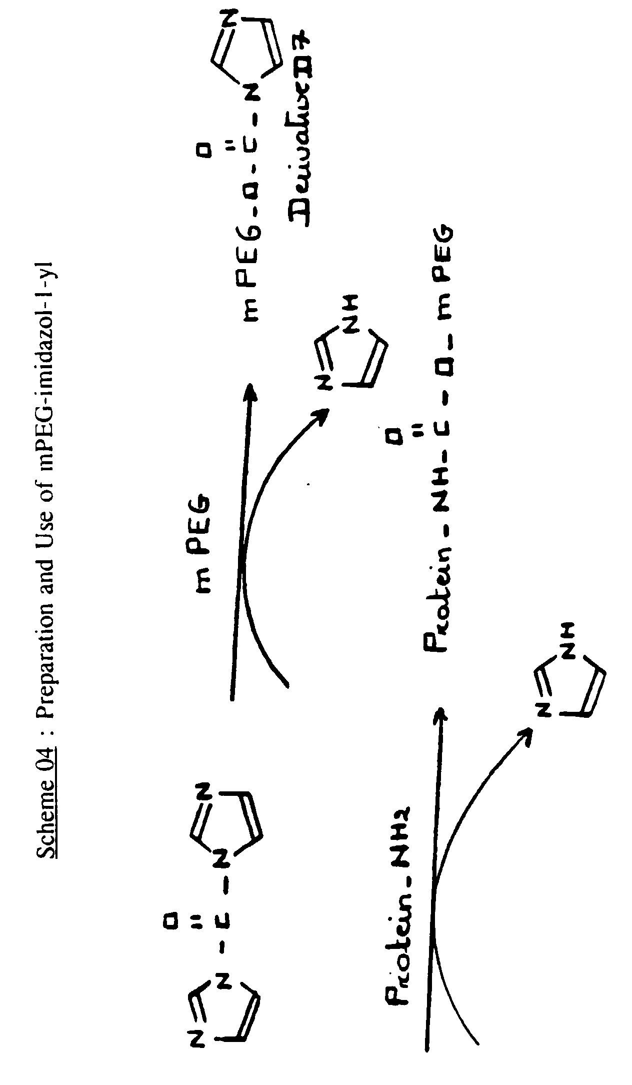

Several versions of a one-step synthesis of derivative D7 using carbonyldiimidazole were reported (C.O. Beauchamp et al, 1983, Anal. Biochem., **131**:25 L. Tondelli et al, 1985, J. Controlled Release, **1**:251.).

Complete transformation of the hydroxyl groups of PEG into derivate D7 was obtained quite recently (J.Brygier et al, 1993, Biotechnol. Appl. Biochem., **15**:in the press.).

Good preservation of activity in the enzymes modified with derivative D7 were usually observed.

Unfortunately, PAG's activated in this manner have a rather mild reactivity, and therefore long reaction times were required for protein modification (48-72 hours, pH 8.5).

The products of protein modification using activated carbonates of PEG (**DERIVATIVES D8, D9** and **10**) also have PEG chains grafted onto the polypeptide amino groups through urethane linkages.

### ACTIVE CARBONATES OF PEG

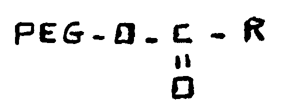

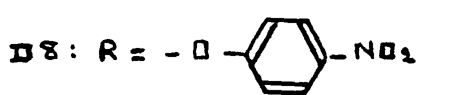

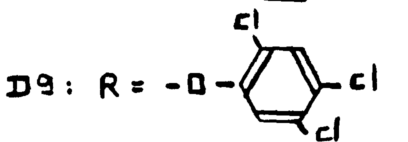

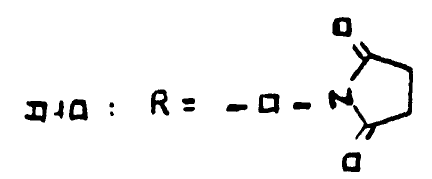

Single-step activation protocols with commercially available chloroformiates of 4-nitrophenol and 2,4,5-trichlorophenol were used for the preparation of derivatives **D8** and **D9**, respectively (E.M. Veronese et al, 1985, Appl. Biochem. Biotechnol., **11**:141.).

These activated PEGs seem to react faster than derivative D7. However, both 4-nitrophenol and 2,4,5-trichlorophenol produced in the course of the PEG-attachment process are toxic hydrophobic molecules with affinities towards polypeptides.

Derivative **10** and a series of related carbonates (in which N-succinimide is replaced by N-phthalimide, N-glutarimide, N-tetrahydrophthalimide and N-norbornene-2,3-dicarboximide) have been recently patented (U.S.Patent 5, 122, 614; June 16, 1992 to S. Zalipsky).

Succinimidyl carbonates of PEG (SC-PEG) constitute a further improvement in urethane-forming PEG reagents.

SC-PEG and its bifunctional analogs (BSC-PEG) of different molecular weights were prepared in a one-pot procedure with an overall yield of 85%.

Polymeric chloroformiate, generated in situ by treatment of PEG with phosgene, was reacted with N-hydroxysuccinimide (HOSu). To estimate the reactivity of SC-PEG, measurements of hydrolysis and aminolysis rates of both activated polymers derived from mPEG-5000 were performed.

The results of these experiments showed that SC-PEG is slightly less reactive yet a more selective reagent. Protein modification reactions with SC-PEG can be performed within short periods of time (± 30 minutes) over a broad pH range, with the highest reactivity at pH 9.3 (U.S. Patent 5, 122, 614; Loc.cit.).

Derivative 10 was also prepared in an other one-pot procedure starting from mPEG (5 kDa) and the commercially available disuccinimidyl carbonate (N. Nijs et al, 1993, Biotechnol. Appl. Biochem., **15** : in the press.). The yield was also 85% and the reactivity of SC-PEG was evaluated using several enzymes including lysozyme, papaya proteinase III, catalase and lactoperoxydase.

The result of these studies showed that only two moles of SC-mPEG per mole of amino group were necessary to achieve high levels (70%) of modification.

Enzyme activities were well preserved. In some cases (papaya proteinase III, catalase), modification with SC-mPEG was accompagnied by an increase of specific activity (250% in the case of papaya proteinase III); (M. Nijs et al.; Loc.cit.).

The HOSu released during polypeptide modification is a non toxic material that is often used in bioconjugate and peptide chemistry as a leaving group residue.

Unlike 4-nitrophenol and 2,4,5-trichlorophenol mentioned above, HOSu does not show affinity toward proteins and thus can be readily removed from the reaction medium by either dialysis or diafiltration or molecular sieving.

**DERIVATIVE 11**, another N-succinimide activated PEG, was patented by K. Iwasaki et al (U.S. Patent 4, 670, 417.; Loc.cit.) and prepared according to **SCHEME 05**.

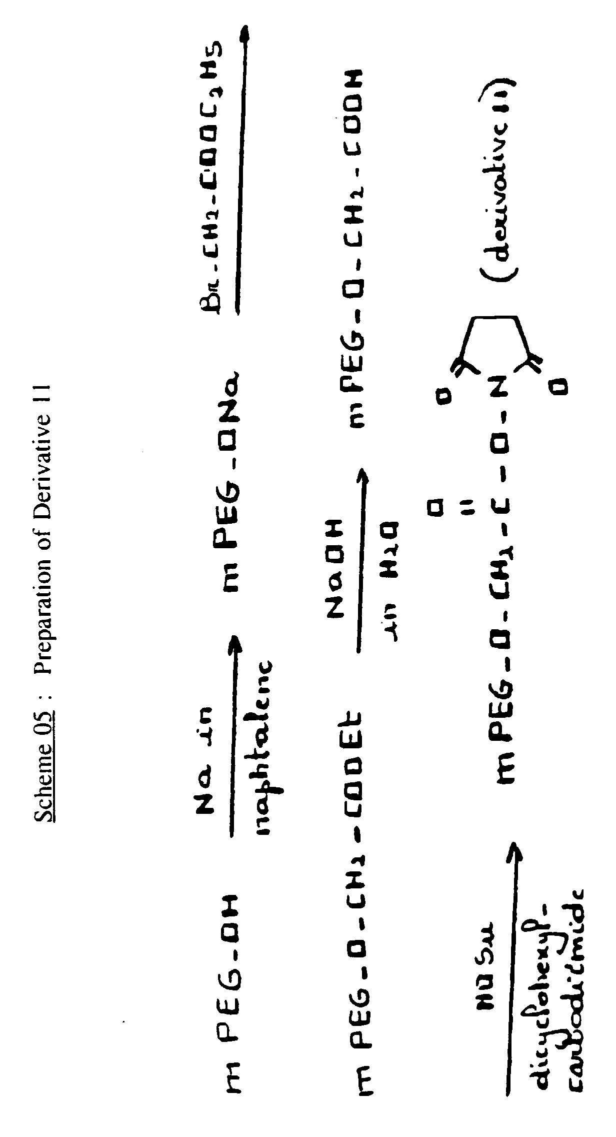

Derivative 11 was prepared by carbodiimide-mediated esterification of carboxymethylated PEG with HOSu.

Carboxymethylation of PEG is one of the most straightforward ways to introduce carboxylic acid end groups onto the PEG polymer.

This is best accomplished, as shown in Scheme 05, by nucleophilic displacement of the bromide in ethylbromoacetate with a PEG alkoxide, followed by saponification of the ester.

An alternative way to essentially the same derivative is by oxidation of the terminal hydroxyls of PEG (G. Johansson, 1986, J. Chromatogr., **368**:309.), but this process is often accompanied by polyether backbone degradation. A number of enzymes were modified using derivative 11 with very good preservation of specific activity (F.M. Veronese et al, 1989, J. Controlled Release, **10**:145.; T. Yashimoto et al, 1986, Biotechnol. Lett., **8**:771.).

The reactivity of derivative 11 was reported to be one order of magnitude higher than those of derivatives reported here so far as was estimated by hydrolysis (t0.5 = 1 minute at pH 7.5,27°C) and aminolysis rates of the activated mPEGs (D.E. Nitecki et al, 1988, in : Peptide Chemistry (T. Shiba and S. Sakakibara, eds) p243, Protein Res. Foundation, Osaka).

Unfortunately, as reported in Scheme 05, preparation of derivative 11 needs 4 steps.

In a variant form of Scheme 05, PEG-carboxymethylazide (**DERIVATIVE 12**) was generated in situ from PEG-carboxymethylhydrazide and then reacted with proteins according to the reaction shown in **SCHEME 06**.

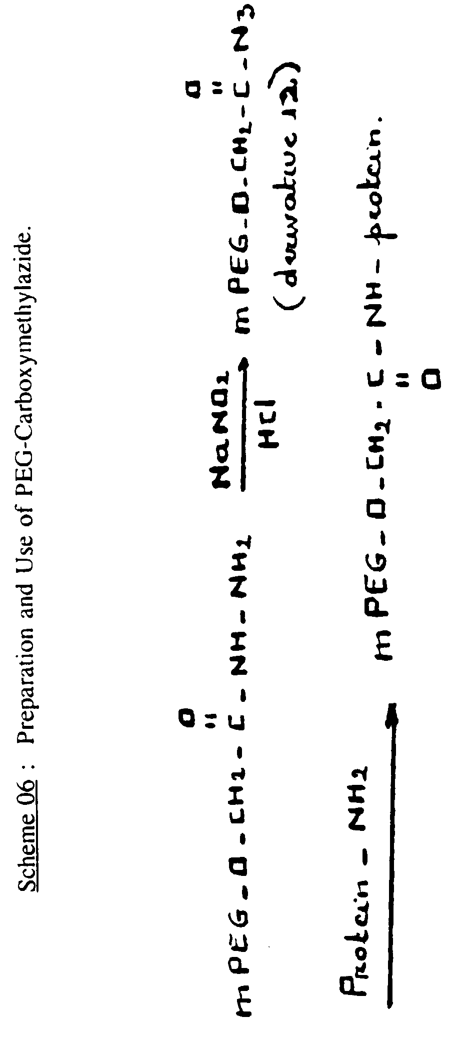

Normally, the slow reacting acylazide functionality gets an additional boost of reactivity due to the stronger electrophilicity of the carboxymethyl residue (U.S. Patent 4, 179, 337, Loc.cit.; U.S. Patent 4,101,380, Loc.cit.; U.S. Patent 4,495,285; 1985 to K. Shimizu, T. Nakahara and T. Kinoshita; U.S. Patent 4,640,835; 1987 to K. Shimizu, T. Nakahara, T. Kinoshita, J. Takatsuka and M. Igarashi).

This variant form which leads to derivative 12 suffers the same drawbacks as the original form which lead to derivative 11.

PEGs have also been converted into **xanthate** (dithiocarbonate) derivatives as **DERIVATIVE 13**, which were shown to be useful for grafting PEG chains onto polypeptides via thionourethane linkages (T.P.King and C. Weiner, 1980, Int. J. Peptide Protein Res., **16**:147.; S. Zalipsky et al, 1987, Int. J. Peptide Protein Res., **30**:740.).

### Structure of derivatives 13

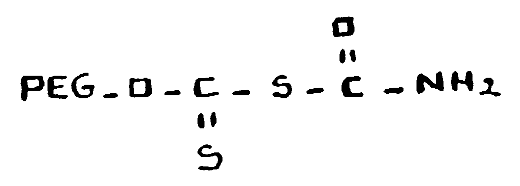

The PEG oxythiocarbonylated proteins conjugates showed good chemical stability in a variety of aqueous buffers. Unfortunately, because of the mild reactivity of derivatives 13, the modification reactions with proteins had to be carried out at elevated pH range (9-10) and for long periods of time (24hrs). Not all polypeptides will resist to such drastic conditions.

Esters of organic sulfonic acids and PEG, p-toluenesulfonates(tosylates) in particular, have been useful as starting materials for the preparation of a variety of functionalized polymers.

Recently, tresylates (2,2,2-trifluoroethanesulfonates) of PEG (**DERIVATIVE 14, mPEG-OSO**_{**2**}**CH**_{**2**}**CF**_{**3**}) were shown to be sufficiently reactive toward amino groups (about 100-fold more reactive than tosylates) to be considered useful as polypeptide modifying reagents (C. Delgado et al, 1990, Biotechnol. Appl. Biochem., **12**:119.).

Unfortunately, mPEG tresylate hydrolyzed readily in aqueous solution. As a consequence, substantial modification of bovine serum albumine at pH 7.5 required up to 16-fold excess of mPEG-tresylate to the amino groups.

This value has to be compared to the 2-fold excess required by the use of SC-PEG (M.Nijs et al, 1993, Loc.cit.).

Modifications with mPEG-tresylate, however, proceeded rapidly since they were complete within 1 hour.

Furthermore, as a result of protein modification with PEG-tresylate, the polymer chains become grafted onto the polypeptides through very stable secondary amine bonds. As a consequence of this, the total charge of the protein does not change as a result of the process of modification.

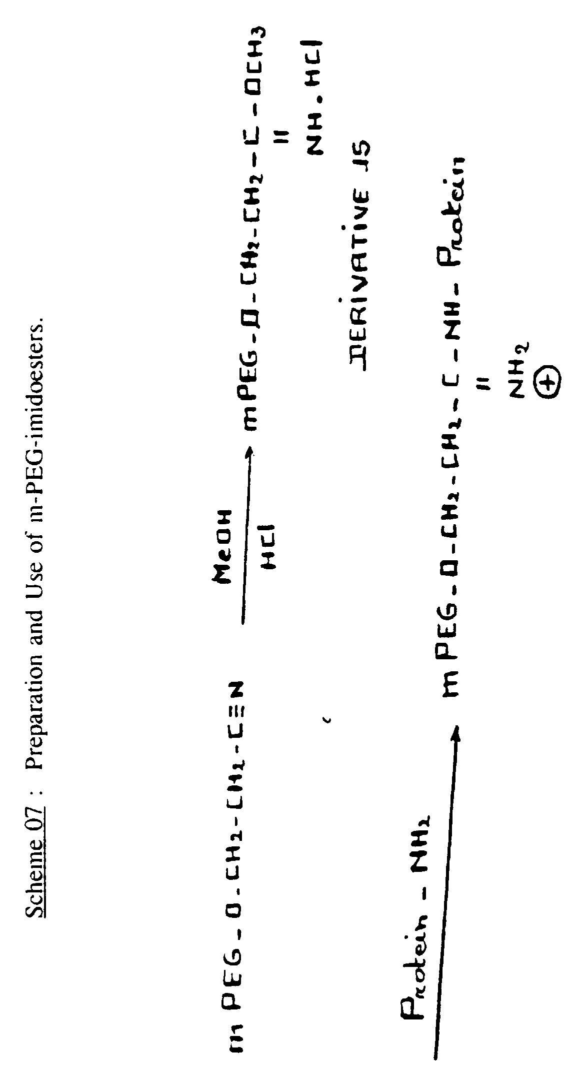

Imidoesters of PEG (**DERIVATIVE 15**) useful for protein modification have been prepared according to the reactions described in SCHEME 07 (Eur.Patent Appl. 0236987 (1987) to H.Ueno and M.Fujino).

As shown in Scheme 07, these activated polymers were prepared by acid-catalyzed methanolysis of mPEG-cyanoalkylethers and reacted with amino groups of proteins at pH 7-9.

An advantage of this approach to protein modification is that the amidinated proteins possess the same net charge as the native ones.

The major drawback of this method is that high excess of imidoesters of PEG were required. Even in these conditions, proteins were modified only to a limited extent.

For example, modification of rIL-2 with 10-fold excess of mPEG-imidoesters to the amino groups resulted in the modification of 2.8 lysylresidues out of the total eleven lysine (Eur. Patent Appl. 0236987, Loc.cit.).

Irreversible modifications of polypeptides by PAG chains were usually selected.

When PAG-polypeptides (as for the conjugates made from derivatives D4 and D5) did not resist hydrolytic cleavage at physiological pH, this was generally considered as a nuisance.

There are three exceptions where reversibility of the modification of peptides and proteins by PAGs was willingly selected.

Garman indeed patented the synthesis of a polymeric analog of dimethylmaleic anhydride (**DERIVATIVE 16**) suitable for PEG attachment to amino groups of polypeptides through an amide linkage which is slowly erodable under physiological conditions.

### Derivative 16

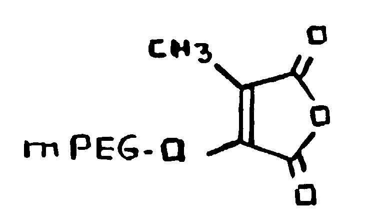

The validity of this approach to give rise to sustained release of active proteins was demonstrated on plasminogen activator proteins.

The PEG-plasminogen activator conjugates obtained by this method yielded active, completely deacylated proteins after 44 hours incubation at pH 7.4 and 37°C (U.S. Patent 4,935,465 (1990) to A.J. Garman).

On the other hand, Glass and co-workers reported preparation of 4-phenoxy-3,5,-dinitrobenzoyl-PEG (J.D. Glass et al, 1979, Biopolymers, **18**:383.). This PEG derivative reacted rapidly and selectively at neutral pH with sulhydryl groups of peptides to yield polymer-peptide adducts in which the components were linked by a thiol-sensitive dinitrophenylene linker. Thus it was possible to attach a peptide to the polymer and, after performing some chemical and/or enzymatic alterations on the conjugate, release the derivatized peptide from the PEG carrier.

PEG-derivatives capable of reacting reversibly, specifically and quantitatively with thiol functions of peptides and proteins have been recently described in a European Patent Application to Y.Looze et al, 1993 which has not been published before the priority date of the present case. Among them, **derivatives 17, 18, 19** and **20** were found to react instantaneously and stoechiometrically by forming PEG-polypeptides conjugates linked through disulfide bonds.

### PEG-derivatives for attachment to thiol functions of polypeptides

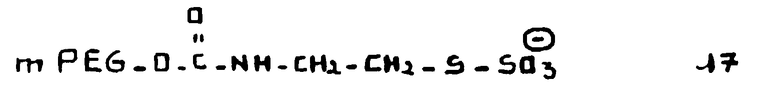

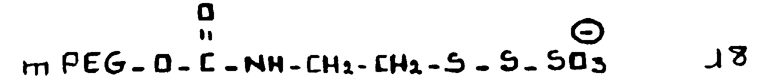

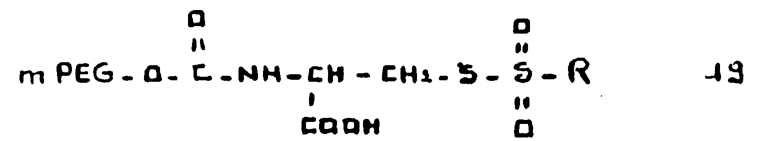

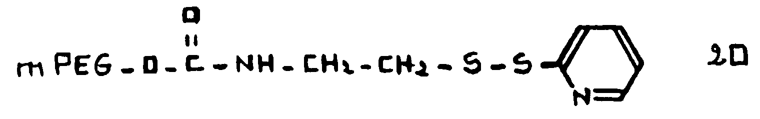

Thus, a series of PAG derivatives have already been prepared and are now available in order to allow the grafting of PAG strands onto polypeptides to facilitate their delivery as drugs or as vehicles to target drugs.

Reversible modification of thiol functions, when naturally present or when introduced into polypeptides may be carried out stoechiometrically (thus by using one mole of PAG-derivative per mole of SH group).

Irreversible modification of the chemical functions present on polypeptides, unfortunately, still suffers from inherent drawbacks, some of which having been briefly outlined here.

Thus, it is obvious that much can still be achieved to find out novel PAG-derivatives with presently unavailable properties.

### SUMMARY OF THE INVENTION.

In the present invention, novel functionalized PAG derivatives were selected for their range of reactivity presently not yet available.

This range of reactivity lies between on one hand, the reactivity of the PAG chloroformiates and, on the other hand, the reactivity of the activated carbonates and esters reviewed previously.

The very high reactivity of the chloroformiates precludes that selectivity and specificity be obtained.

Thus chloroformiates are capable to readily react with amines, alcohols, phenols, carboxylic acids, amides... .

On the other hand, most of the PAG derivatives previously reviewed, show selectivity towards amines.

This means that most of them will react with amines and will not react with other chemical functions such as alcohols.

Unfortunately, these PAG derivatives have a poor reactivity as compared to that of the chloroformiates.

When use is made of polyfunctional compounds such as e.g. : peptides, modification of their amino functions by a non selective acylating agent requires that the other functions be first protected.

After the acylation step, deprotection may then be carried out.

Thus, the use of a non selective acylating agent requires the need for additional reaction steps which complicates the overall process.

On the other hand, the use of a less reactive acylating agent may seriously impair the yield of the reaction and further requires that additional purification steps be included in the overall process.

Thus, there is an urgent need to find out quite novel PAG derivatives with both high reactivity and high selectivity towards amine functions.

It was discovered here, in the present invention that certain PAG carbamates fulfilled the purpose in view. The carbamates are represented by the general formula 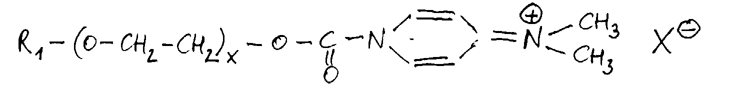 wherein R₁ is H or CH₃, x is an integer between 10 and 1000 and X^{⊖} is an anion.

As a typical example of these PAG carbamates, one may cite the case of mPEG-N-(4-dimethylamino)-pyridine carbamate whose structure is :

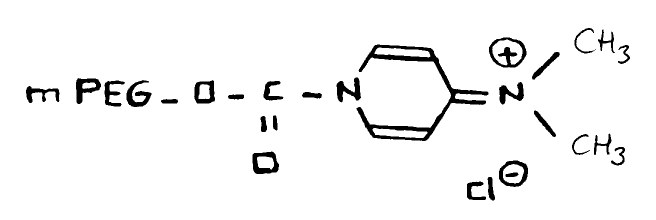

The electron withdrawing effects of the group of atoms, here, in position para, with respect to the carbamate function, is responsible for the high reactivity of this mPEG-derivative, which reacted stoechiometricaly and instantaneously with amines giving quantitative acylation yields.

On the other hand, their selectivity towards amines was evaluated and found quite satisfactory.

### DESCRIPTION OF THE INVENTION

Accordingly, in the present invention, novel PAG derivatization routes were investigated to find out new PAG-derivatives able to achieve the chemical modification of the amino function of amine-containing compounds under conditions and with advantages presently unavailable.

According to the present invention, these new PAG-derivatives may be built up starting with any PAG such as :
- polyethylene glycol with general formula

   HO-(CH₂-CH₂-O)ₓ-CH₂-CH₂-OH

   (where x is an integer as defined above) and
- monomethoxy polyethylene glycols with general formula

   CH₃O-(CH₂-CH₂-O)ₓ-CH₂-CH₂-OH

   (where x is an integer as defined above).

The ®Pluronics are typical examples of such copolymers.

Thus, the new PAG-derivatives described in the present invention possess a great diversity of structure.

The preference, however, has been given to those which are polymeric by nature.

On the other hand, the functionalized PAs, described here, have in common, an activated carbamate function.

A typical example of such an activated carbamate function is disclosed in example 1.

Example 1, which described a method for the obtention of an activated carbamate function merits to be briefly commented.

In this example 1, mPEG-N-(4-dimethylamino)-pyridine carbamate (D) was quantitatively obtained after reacting stoechiometric amounts of mPEG-chloroformiate and 4-dimethylaminopyridine.

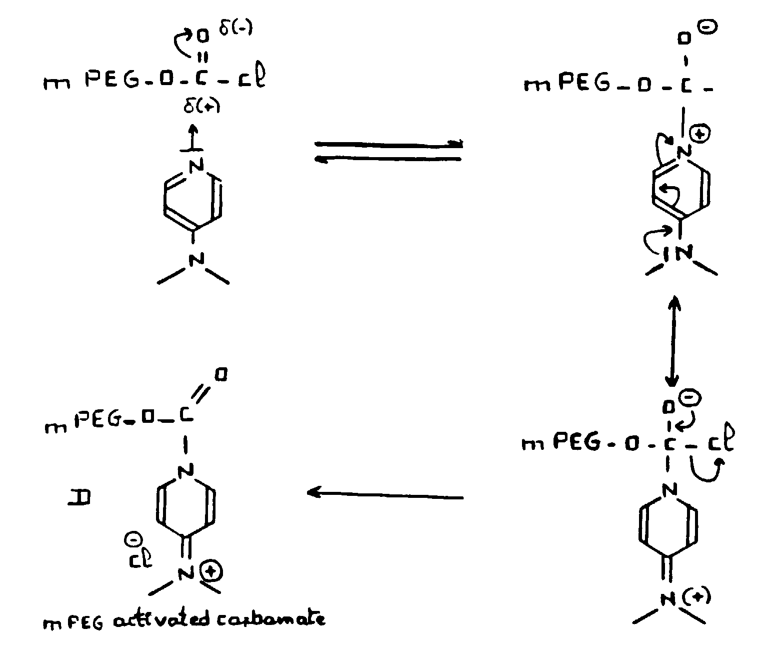

In the expected mechanism, as shown above, the nucleophilic attack on the mPEG-chloroformiate by 4-dimethylaminopyridine constitutes the first step.

This seems reasonable since pyridine is well-known to catalyse acylations by chloroformiates by forming with the latter ones unstable complexes.

In the example commented here, it is expected that such a complex might be stabilized by the resonance interactions as shown above.

On the other hand, the carbamate which formed in example 1 was found to be particularly reactive, as compared to e.g.: mPEG-imidazol-1yl-carbamate previously commented (see background of the invention).

It is expected that this unusual reactivity might be due to electron withdrawing effects not encountered in mPEG-imidazol-1yl-carbamate.

The activated carbamate of example 1 was even found much more reactive than the activated carbonates or the activated esters reviewed in the paragraph "background of the invention ".

As compared to mPEG chloroformiates, the activated carbamate D shows a lesser reactivity.

While mPEG chloroformiates readily react with amines, alcohols, phenols, carboxylic acids ..., the activated carbamate discovered here reacts with the sole amines.

This high degree of selectivity thus affords the opportunity to modify amino functions in polyheterofunctional compounds without requiring to protect the other chemical functions.

This property, illustrated in examples 2, 3 and 4, represents an important advantage.

The possibility to selectively modify amino functions in the presence of other chemical functions is of prime interest in various industrial fields.

Among these industrial fields, the biotechnological and the pharmaceutical industries, particularly, make use of polyheterofunctional compounds such as nucleotides, nucleosides, carbohydrates, peptides,... . For such industries thus, the high degree of selectivity shown by the activated carbamates discovered here, might thus be of primary interest.

In addition to selectivity, the activated carbamates discovered here show high reactivity.

High yields of acylation are obtained by using stoechiometric amounts of reactants. In most cases, acylation proceeds instantaneously.

### EXAMPLES

### Example 1 : SYNTHESIS AND PRELIMINARY CHARACTERIZATION OF POLYETHYLENE GLYCOL-N'-(4-DIMETHYLAMINO)-PYRIDINE CARBAMATE. (Derivative "D" or mPEG-N(DMA)Py)

A solution is prepared from mPEG (25g; 5 mmoles) and toluene (250 ml). Traces of water present in mPEG (0.2 % according to the Karl Fischer titration) are removed by the azeotropic distillation of 150 ml toluene. The remaining is then rotaevaporated to dryness and the residue redissolved in acetonitrile (100 ml) at 0°C. Phosgene (20 mmoles; 10 ml of a 2M solution in toluene) is added to the cold mPEG solution.

The temperature of the reaction mixture was allowed to increase up to room temperature and the reaction proceeds for 20 hours. Phosgene in excess is then eliminated under vacuum and thereafter, the solution is rotaevaporated in order to remove HCl.

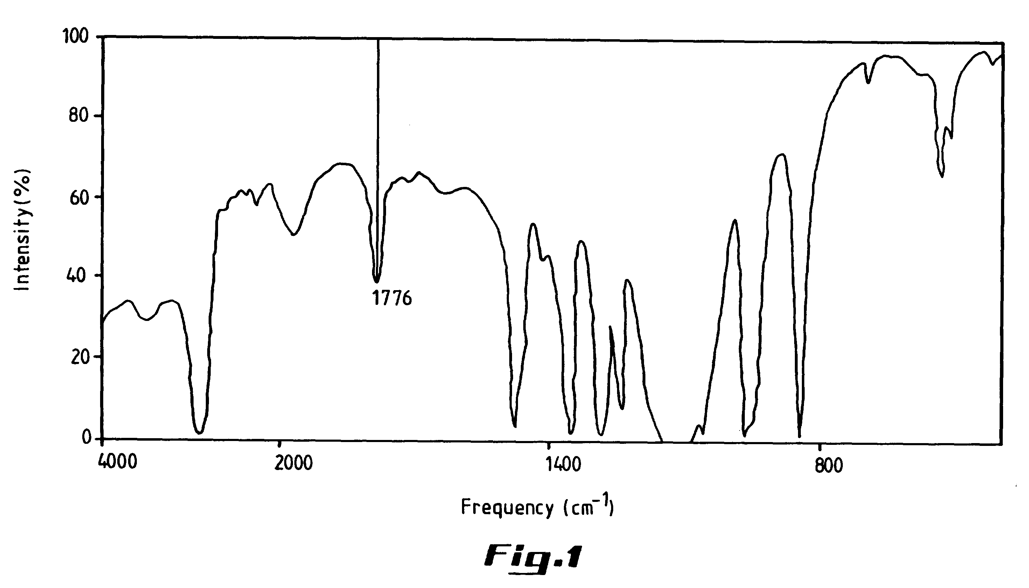

An aliquot was removed for analysis. The Infrared Spectrum (Figure 1.1) showed an absorption band at 1776 cm⁻¹, characteristic of the C=O vibrations of chloroformiates (M. Matzer, R.P. Kurkji and R.J. Cotter, 1964, Chem. Rev., **64**:645.).

From the absence of absorption band in the region between 1715 and 1740 cm⁻¹, it is concluded to the absence of formation of di(mPEG)carbonate.

The yield of obtention of mPEG chloroformiates was shown to be quantitative on the basis of the results of Cl⁻ titration using silver nitrate as the titrant (H.G. Ashbrun, A.R. Collet and C.L. Lazzell,1938, J. Am. Chem. Soc., **60**:2933.).

The mPEG chloroformiate is redissolved in cold acetonitrile (100 ml; 0°C). To this solution, solid 4-dimethylaminopyridine (5.2 mmoles) is added which results in the instantaneous conversion of mPEG chloroformiate into mPEG-N1-(4-dimethylamino) pyridine carbamate. The yield, 100%, is established as follows:

An aliquot of the solution of **D** in acetonitrile was removed from the bulk and added dropwise to dried diethylether. Solid **D** was collected by filtration, washed with diethylether and dried in vacuo over P₂O₅.
Figure 1. Infrared Spectrum of mPEG chloroformiate

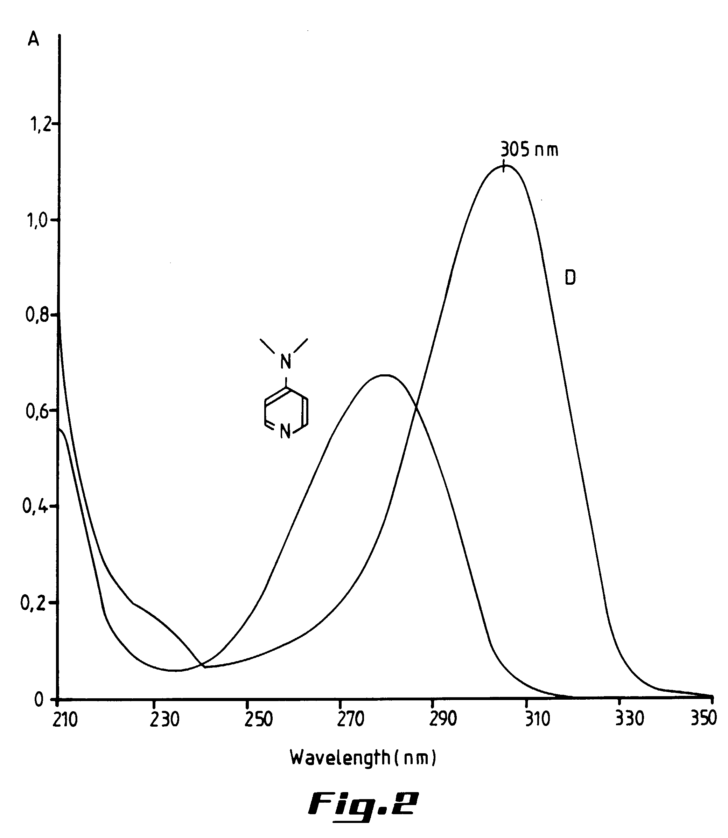

The UV-spectrum of a freshly prepared solution of known concentration of **D** in acetonitrile was recorded. The UV-spectrum of **D**, displayed in **Figure 2**, showed an absorption band at 305nm (8,11 = 24100 M⁻¹.cm⁻¹) where mPEG was transparent. Upon hydrolysis, this spectrum converted into another one (also shown in Figure 2) which was shown to be identical to that of authentic 4-dimethylaminopyridine (λₘₐₓ = 276nm; ε₂₇₆ = 13900 M⁻¹.cm⁻¹).

Using the value ε₂₇₆ = 13900 M⁻¹.cm⁻¹ for 4-dimethylaminopyridine, it was calculated that hydrolysis of **D** gave rise to 1.02 ± 0.03 mole 4-dimethylaminopyridine per mole D1.

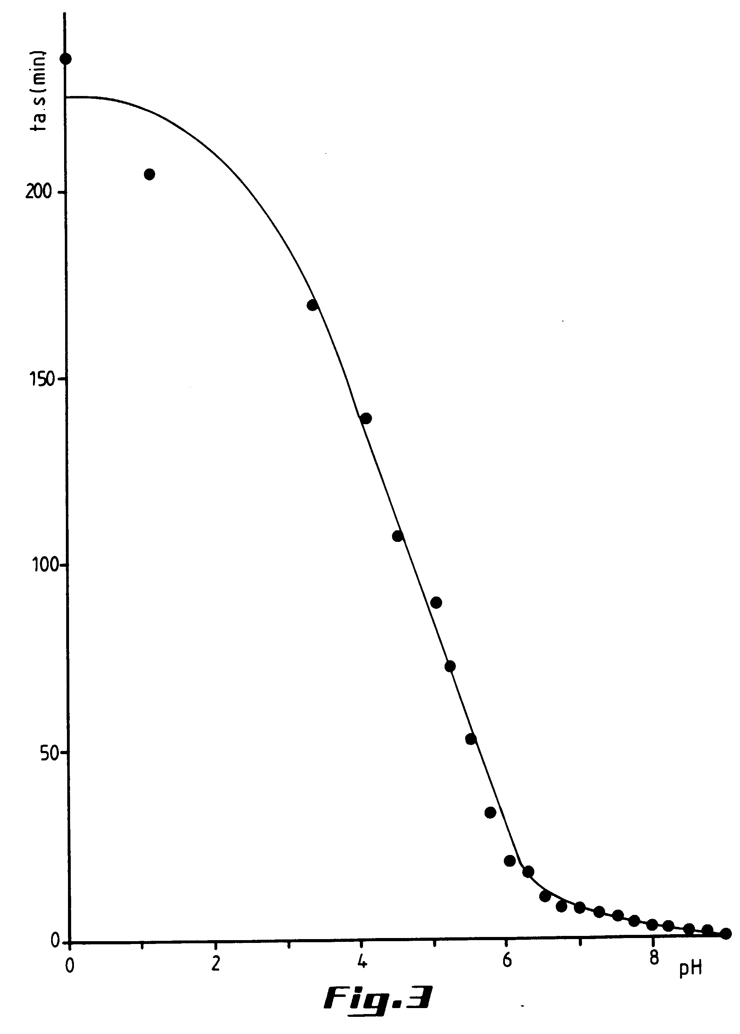

As expected, **D** is quite reactive including towards water. As shown in **Figure 3** hydrolysis proceeds more slowly at acidic pHs than in neutral or slightly alkaline media. This observation, possibly, may indicate that the nucleophilic attack by OH is the rate limiting step of the hydrolysis process.

In any case, **D** is better conserved as a solution in e.g.acetonitrile rather than as solid powdered forms.
Figure 2. UV-absorption spectra of D (in acetonitrile) and of its UV-absorbing hydrolysis product identified as 4-dimethylaminopyridine.
Figure 3. pH-dependence for kinetics of hydrolysis of D. A stock solution of D in acetonitrile was diluted in 50 mM buffers at appropriate pH. A₃₁₅ was recorded continuously, at 25°C, as a function of time and t_{0.5} determined. Dots (●) represent experimental values.

### Example 2 : REACTIVITY OF mPEG-N-(DMA)Py IN WATER CONTAINING SOLVENTS : AMINOLYSIS VERSUS HYDROLYSIS.

Since **mPEG-N-(DMA)Py** showed to be readily hydrolyzed, the necessity to perform aminolysis in strictly anhydrous conditions needed to be examined.

This information was provided by comparing the relative rates of hydrolysis and aminolysis.

Practically, we thus determined the yield of obtention of **DERIVATIVE 20** synthesized, according to SCHEME 2.1., in presence of water.

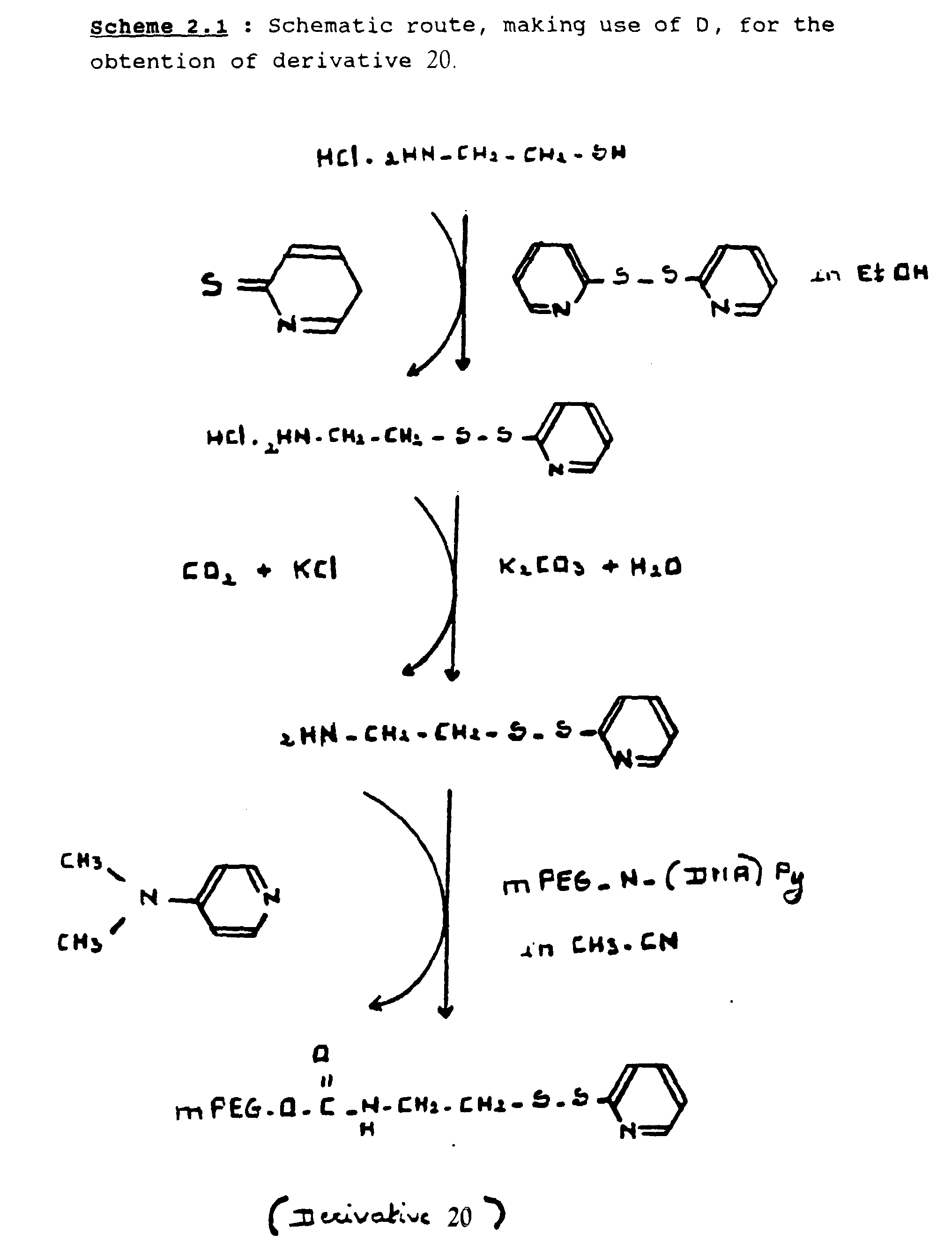

Thus, a solution of cysteamine hydrochloride (2.2 mmoles) in **EtOH** (10 ml) was added dropwise to a solution containing 20 mmoles of 2,2'-dipyridyldisulfide in **EtOH** (30 ml). After the addition was complete, 10 ml of an aqueous solution of **K**_{**2**}**CO**_{**3**} (1.1 mmole) was added and the resulting mixture was submitted to reaction with 2 mmoles of **mPEG-N-(DMA)Py** dissolved in acetonitrile (40 ml). The reaction mixture was maintained under stirring for 2 hours at room temperature, concentrated by rotaevaporation and then added, dropwise, to diethylether (300 ml).

Derivative 20 was collected by filtration, washed with diethylether and dried in vacuo over P₂0₅. The yield was 10 g (96 % of theory).

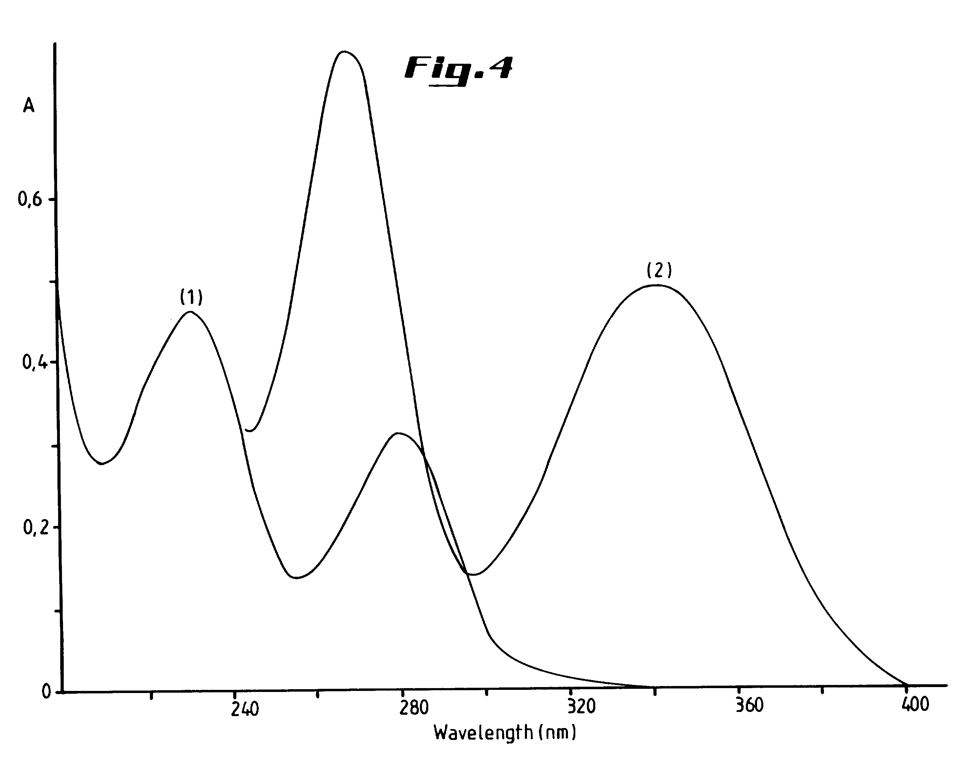

Figure 4. illustrates the UV absorption spectrum of an aqueous solution of derivative 20 which displayed maxima at 231 and 281nm (ε₂₈₁=4900 M⁻¹.cm⁻¹).

When cysteine was added in excess, 2-thiopyridone was liberated from derivative 20 and an absorption band at 343nm appeared. I determined a value for ε₃₄₃ = 7725 M⁻¹.cm⁻¹ in excellent agreement with the value (ε = 7700 M⁻¹.cm⁻¹) from the literature.

Figure 4.: UV-absorption spectrum of derivative 20 (0.06mM, 25°C, pH 6,H20) before (1) and after (2) the addition of 2-cysteine (5mM).

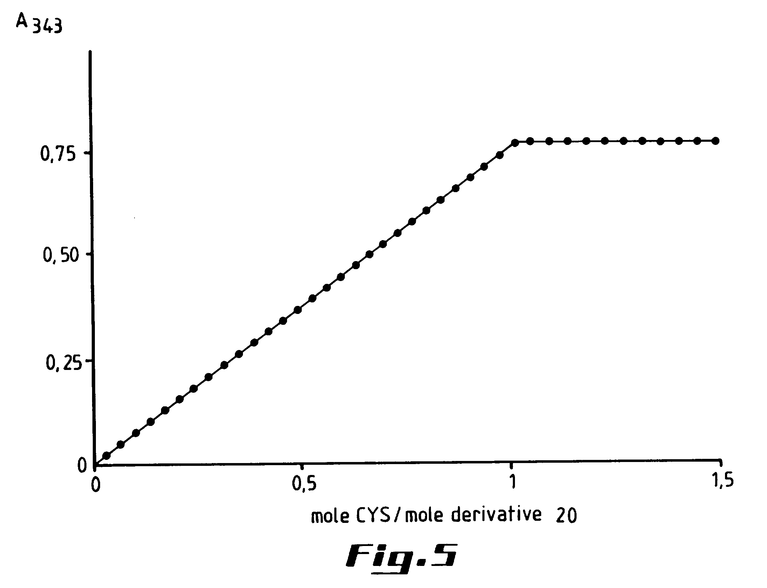

Furthermore, the spectrophotometric titration of derivative 20 with freshly prepared and standardized (with 2,2'-dipyrridyldisulfide) solutions of cysteine confirmed the stoechiometry of the reaction as shown in **Figure 5**.

I thus concluded that derivative 20 could be quantitatively prepared from mPEG-N-(DMA)Py even in the presence of an excess of water over the amino compound (as in the experiment reported here wherein the excess was 250 moles H₂O/mole NH₂).

In other words, we concluded that derivatization of amino-containing compound by mPEG-N-(DNA)Py does not require strictly anhydrous conditions.

Figure 5. : Spectrophotometric titration of an aqueous 0.1mM solution of derivative 20 by standardized 10mM L-Cysteine solution. The release of 2-thiopyridone from derivative 20 was measured at 343nm and 25°C. Dots (●) represent experimental values.

This represents an advantage when compared to methods making use of derivatives such as the mPEG chloroformiate. This may represent a decisive advantage when derivatizing a hygroscopic amino-containing compound.

Taking into account the enormous difficulties encountered when attempting to fractionate mPEG (resulting from hydrolysis of mPEG-N-(DMA)Py) from the mPEG conjugate one should properly appreciate this advantage.

This example, furthermore, provides a guarantee that a molecule which would contain both amino and hydroxyl functions may be N-acylated specifically and selectively.

### Example 3 : RELATIVE REACTIVITIES OF D TOWARDS PRIMARY AND SECONDARY AMINES.

To examine the relative rates of reactivity of D towards primary and secondary amines, D (0.75 mmole in 15 ml acetonitrile) was allowed to react with 4-amino-2,2,6,6-tetramethylpiperidine (0.825 mmole).

The precipitate which formed, in the course of the instantaneous reaction, was eliminated by filtration.

The filtrate was concentrated by rotaevaporation and added, dropwise, to diethylether and dried in vacuo over P₂O₅.

An aqueous solution of D.3.1 (1.2 mM) was prepared and shown to be transparent in the UV-region between 200 and 350 nm which observation indicated that 4-dimethyl aminopyridine was indeed quantitatively substituted by 4-amino-2,2,6,6-tetramethylpiperidine.

To examine whether the primary or the secondary amine function, contained in 4-amino-2,2,6,6-tetramethylpiperidine substituted for 4-dimethylaminopyridine, derivative D.3.1. was reacted with fluorescamine, known to give rise to fluorescent adduct compounds with primary amines and not with secondary amines.

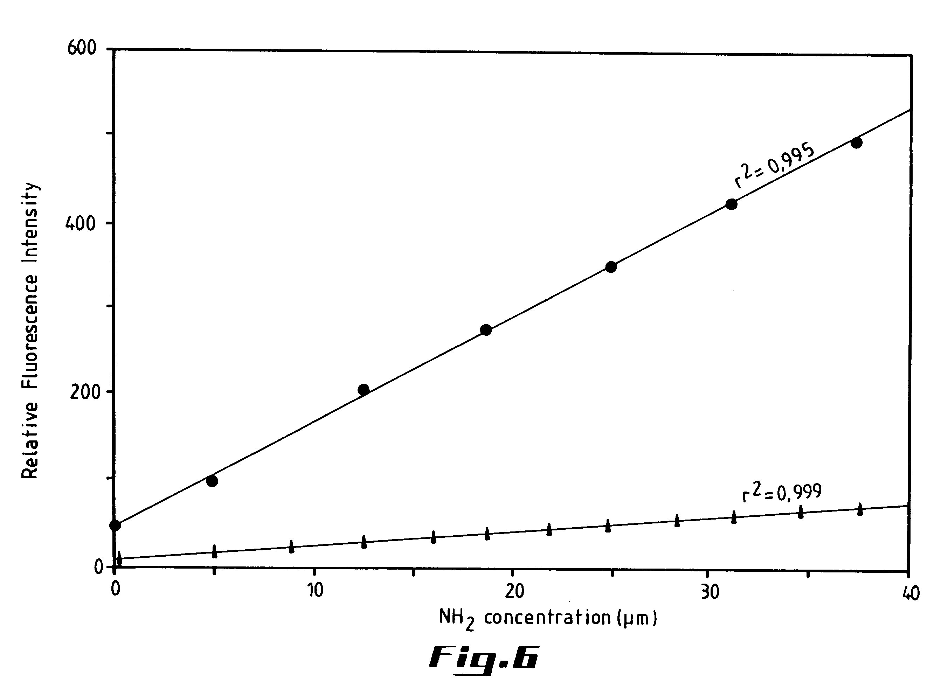

The results of this assay are displayed in **Figure 6**.

From these results, it was calculated that 94%, at least of D.3.1. corresponded to the structure **3.1.A** and less than 6% to the structure **3.1.B**.

### Structures of D.3.1.

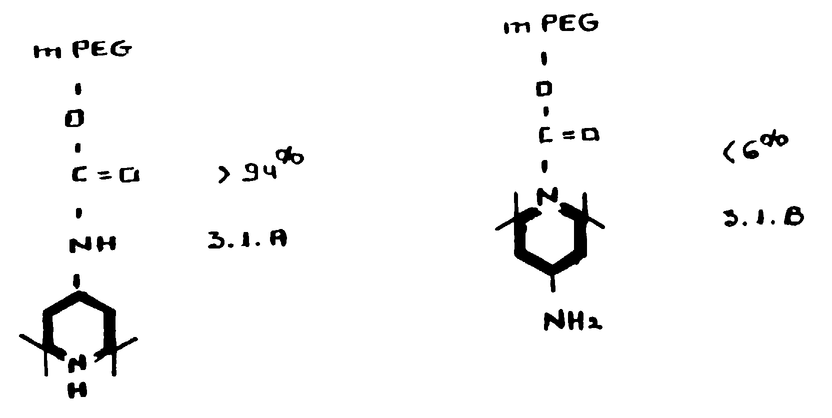

Figure 6. Fluorescamine assay of 4-amino-2,2,6,6-tetramethylpiperidine (●) and of derivative D.3.1.(*). Dots (●) and (*) represent experimental values.

### Example 4 : REACTIVITIES OF D AND OF mPEG CHLOROFORMIATE TOWARDS PHENOLS.

To examine this point, mPEG chloroformiate was allowed to react, at the same time, with equimolar amounts of a phenol and of 4-dimethylaminopyridine. The products, in the reaction mixture, were then fractionnated and analyzed separately. p-dimethylsulfoniophenol was choosen, arbitrarily, as the archetype of the phenolic compounds.

### Reactivities of mPEG chloroformiate and D towards p-dimethylsulfoniophenol.

To a chilled solution (0°C) of mPEG chloroformiate (5mmoles) in acetonitrile (100ml) prepared as quoted in example 1, p-dimethylsulfoniophenol (1.33g; 5 mmoles) was added and allowed to dissolve (10 minutes).

4-dimethylaminopyridine (613 mg; 5.1 mmoles) was then added and the reaction(s) allowed to proceed for 3 hours at room temperature.

The precipitate which formed in the course of the reaction(s) was collected by filtration, washed with acetonitrile and dried in vacuo over P₂O₅ to constitute the first fraction (**F1**).

The clear filtrate, on the other hand, was concentrated by rotaevaporation and added dropwise to diethyl ether and dried in vacuo over P₂O₅ to constitute the second fraction (**F2**).

**F1** contained 925 mg of a substance which was identified to as p-dimethylsulfoniophenol(methylsulfate salt) on basis of :
- its UV absorption spectra in protonated and unprotonated forms
- the value of its pKa
- the value of its molecular weight calculated from the results of the titration (with NaOH) of F1 solution of known massic concentration.

On the other hand, **F2** (24.70 g of material) contained a mixture (± 50 % each) of D and of hydrolyzed D (mPEG + 4-dimethylaminopyridine)

### Synthesis of mPEG-(p-dimethylsulfonio)phenyl carbonate

This compound was obtained (yield 65%) from mPEG chloroformiate and p-dimethylsulfoniophenol in the presence of one equivalent of triethylamine.

From all these results, it may be concluded that :
- 4-dimethylaminopyridine reacted much faster with mPEG chloroformiate than a phenol, typified here by p-dimethylsulfoniophenol, did.
- under the experimental conditions used here, the reaction (if any) between D and a phenolic function was quite slow.

## Claims

1. A compound having the structure : 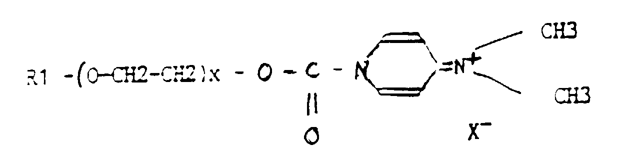 wherein R1 is H- or -₃HC, x is an integer between 10 and 1000 and X⁻ is an anion.

2. A compound according to claim 1 wherein X⁻ represents Cl⁻.

## Patentansprüche

1. Verbindung mit der Struktur: 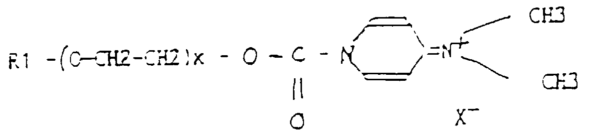 in der R1 H- oder CH₃- ist, x eine ganze Zahl zwischen 10 und 1000 und X⁻ ein Anion ist.

2. Verbindung nach Anspruch 1, inder X⁻ Cl⁻ bedeutet.

## Revendications

1. Composé ayant la structure : 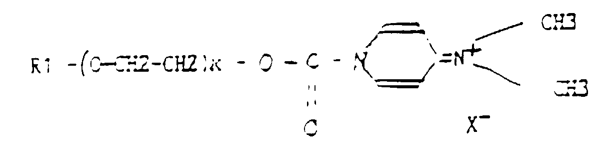 dans laquelle R1 est H- ou -₃HC, x est un nombre entier entre 10 et 1000 et X⁻ est un anion.

2. Composé suivant la revendication 1, dans lequel X⁻ représente Cl⁻.
